# EUROPEAN PATENT SPECIFICATION

(11) **EP 1 378 753 B1**
(45) Date of publication and mention of the grant of the patent: **31.05.2006**
(21) Application number: 02014542.1
(22) Date of filing: 01.07.2002
(51) Int. Cl.: G01N 33/68, A61K 31/12

(54) **A screening method and compounds for treating Friedreich Ataxia**
Screeningverfahren und Verbindungen zur Behandlung von Friedreich ataxia
Procédé de criblage et composés destinés au traitement de la maladie de friedreich

(43) Date of publication of application: 07.01.2004
(73) Proprietor: Santhera Pharmaceuticals (Schweiz) GmbH, 4410 Liestal (CH)
(72) Inventor: Meier Thomas, 4056 Basel (CH); Jauslin, Matthias, 4056 Basel (CH); Schoumacher Fabrice, 68440 Eschentzwiller (FR)
(74) Representative: Grünecker, Kinkeldey, Stockmair & Schwanhäusser Anwaltssozietät

(56) References cited:
- US-A- 5 321 138
- US-A- 6 133 322
- FRYER M J: "Rationale for clinical trials of selenium as an antioxidant for the treatment of the cardiomyopathy of Friedreich's ataxia." MEDICAL HYPOTHESES, vol. 58, no. 2, February 2002 (2002-02), pages 127-132, XP001095763 ISSN: 0306-9877
- PILCH JACEK ET AL: "Friedreich's ataxia." JOURNAL OF CHILD NEUROLOGY, vol. 17, no. 5, May 2002 (2002-05), pages 315-319, XP001095762 ISSN: 0883-0738
- LODI R ET AL: "Mitochondrial dysfunction in Friedreich's ataxia: from pathogenesis to treatment perspectives." FREE RADICAL RESEARCH. SWITZERLAND APR 2002, vol. 36, no. 4, April 2002 (2002-04), pages 461-466, XP001097344 ISSN: 1071-5762
- RUSTIN PIERRE ET AL: "Heart hypertrophy and function are improved by idebenone in Friedreich's ataxia." FREE RADICAL RESEARCH. SWITZERLAND APR 2002, vol. 36, no. 4, April 2002 (2002-04), pages 467-469, XP001097343 ISSN: 1071-5762
- SCHULZ J B ET AL: "Oxidative stress in patients with Friedreich ataxia." NEUROLOGY, vol. 55, no. 11, 12 December 2000 (2000-12-12), pages 1719-1721, XP001095770 ISSN: 0028-3878

## Description

### Field of the invention

The present invention relates to a method for identifying and/or validating candidate substances for the treatment of Friedreich Ataxia (FRDA). Furthermore, the present invention relates to the use of Glutathione peroxidase (GPX) mimetics for the preparation of a medicament for the treatment of FRDA. Another aspect of the present invention relates to the use of cells with reduced frataxin expression for identifying and/or validating candidate substances for the treatment of Friedreich Ataxia.

### Background of the invention

Friedreich Ataxia (FRDA) is the most prevalent inherited ataxia, with a frequency of 1 in 50.000 individuals. This progressive neurodegenerative disorder is an autosomal recessive disease. Friedreich Ataxia results from the reduced expression of frataxin, a nuclear encoded mitochondrial protein. It is a neurodegenerative disease characterized among other symptoms by progressive gait and limb ataxia, decreased vibration sense, and muscular weakness of the legs. Hypertrophic cardiomyopathy is present in most patients. At the cellular level, the lack of frataxin leads to an increased oxidative stress that causes cell damage. Onset of symptoms usually takes places in early childhood and typically before 25 years of age. Currently, there is no treatment available.

Helveston et al. (Movement Disorders, Vol. 11, 1996, 106-107) suggests that antioxidant compounds or compounds that function as sulfhydryl group donors to replenish glutathione may be helpful in slowing disease progression in FRDA by compensating the body's inability to compensate increased oxidative stress.
However, Rustin et al. (The Lancet, Vol. 354, 1999, 477-479) suggests that the application of antioxidants, such as ascorbate or glutathione, might actually be harmful because they reduce the mitochondrial iron overload from the Fe³⁺ to the Fe²⁺ oxidation state, thereby catalysing more oxygen radical formation.
Furthermore, at present there is no cell culture model with a disease relevant phenotype available that would allow to screen and identify potential drug candidates for the treatment of FRDA.
Because of the lack of treatment available for FRDA and the lack of an FRDA relevant bioassay, there is a need for effective medicaments as well as a need for a tool for identifying and/or validating compounds for treating FRDA.

### Disclosure of the invention

One aspect of the present invention provides a new method for identifying and/or validating candidate substances for the treatment of Friedreich Ataxia, comprising the steps of
a) providing cells with reduced frataxin expression,
b) incubating the cells of step a) in selenium-restricted medium,
c) reducing the cellular glutathione content of the cells of step b),
d) contacting the cells of step c) with a candidate substance, and
e) evaluating the response of the cells of step d),
wherein steps b), c) and d) may be also be performed in any other order than b), c) and d), the order b), c) and d) being preferred.

It is preferred to perform step b) before steps c) and/or d) because the depletion of selenium in the cells generally requires some time. It is also preferred to reduce the cellular glutathione content before contacting the cells with a candidate substance. The skilled person realises that the steps b), c) and d) may be also be performed in any other order, such as, e.g. c), b) and d) or c), d) and b).

"Cells with reduced frataxin expression" as defined herein are any cells isolated or derived from a mammalian subject that expresses frataxin at a reduced level in comparison to cells from the same healthy mammalian species, preferably at an at least 50 % reduced level, more preferably at an at least 80 % reduced level and most preferably at an at least 95 % reduced level. While in some cases such frataxin deficient cells may be isolated from a mammalian subject, cells with a reduced frataxin expression may also be derived by manipulating normal cells by techniques known to those in the art such as antisense technology, DNA decoys for DNA-binding factors that regulate frataxin expression, molecular engineering, for example altering or deleting part or all of the frataxin gene or its regulatory DNA-sequences, and the like.

Preferably, said cells are isolated or derived from humans. More preferably, these cells are derived or isolated from Friedreich Ataxia (FRDA)-patients, most preferably these cells are fibroblast cells derived or isolated from Friedreich Ataxia (FRDA)-patients.

"A reduced cellular glutathione content" according to the invention as used herein is defined as at least a 1.3-fold reduction, preferably a 2-fold reduction, more preferably more than a 2.5-fold reduction.

The term "selenium-restricted medium" as used in the context of the present invention means that the culture medium does not contain exogenous selenium. Preferably the only selenium source is Fetal Calf Serum. More preferably the medium is a chemically defined serum-free medium that does not contain any selenium at all.

In a preferred embodiment the cellular glutathione content of frataxin reduced cells is reduced by inhibiting the *de novo* synthesis of glutathione. More preferably, the cellular glutathione content is reduced by the addition of an inhibitor of the γ-glutamyl cysteine synthetase, most preferably by the addition of BSO (L-buthionine-(S,R)-sulfoximine).

It was surprisingly found that fibroblasts from Friedreich Ataxia patients, when cultured under selenium-restricted conditions, exhibit a specific sensitivity towards endogenous cellular stress generated by the inhibition of *de novo* glutathione synthesis. Under these conditions, FRDA fibroblasts die rapidly, in contrast to fibroblasts from control donors, which are considerably less sensitive to this treatment. This sensitivity of FRDA fibroblasts towards endogenous stress was confirmed with several unrelated FRDA fibroblasts cell lines. In the case of Friedreich Ataxia, this is the first cellular model using mammalian cells that display a disease-relevant phenotype.

The response to be evaluated in the method of the invention is preferably an increased plasma membrane permeability and/or cell death. Cell death and plasma membrane permeability may be measured according to methods known to the skilled artisan, such as for example referred to in Darzynkiewicz Z. et al. "Cytometry in Cell Necrobiology: Analysis of Apoptosis and Accidental Cell Death (Necrosis)". Cytometry 27, 1997,1-20.

In a preferred embodiment, the method according to the invention comprises an additional control scenario to establish FRDA-related specificity of the compound's physiological activity. Preferably, the response in the method of the invention is compared to the response of control cells with normal frataxin expression and/or normal cellular glutathione content and/or under selenium-supplemented incubation conditions to said same candidate substance. All cells that comprise frataxin and/or glutathione at a normal cellular level as well as those cells that are grown in selenium-supplemented medium are "live" control cells. An unspecific or cytotoxic candidate will kill both, the test and the control cell, independent of the level of frataxin, glutathione or selenium in those cells, while an FRDA-positive candidate substance will lead to the survival of both, the control and the test cells.
An inactive non-toxic compound will result in killing of the test cells but survival of the control cells.

In a further preferred embodiment, said response in the method of the invention is compared to the response of control cells with reduced frataxin expression and reduced cellular glutathione content, which are grown in selenium-restricted medium, to a known effective candidate substance, preferably compared to the response of FRDA-fibroblasts, which are reduced in cellular glutathione content, to Idebenone (6-(10-hydroxydecyl)-2,3-dimethoxy-5-methyl-1,4-benzoquinone), or Ebselen (2-phenyl-1,2-benziso-selenazol-3-(2H)-one).

Using the method of the invention, it is demonstrated that Idebenone can rescue FRDA cell death, indicating that this cell model is appropriate for identifying and validating candidate compounds for treatment of this disease. The cell model of the present invention also provides the first real proof of concept (validation at the biochemical and cellular level) that ldebenone is indeed suited to treat FRDA patients.

Also, several further antioxidants were also found to counteract the FRDA cell death phenotype.

It has therefore been demonstrated that the cellular assay according to the invention displays an FRDA disease-relevant phenotype.

The cell culture model of the present invention relies on the increased sensitivity of frataxin reduced cells towards oxidative stress. This phenomenon is linked to glutathione metabolism since depleting cellular glutathione (GSH) with an inhibitor of γ-glutamyl cysteine synthetase, such as L-buthionine-(S,R)-sulfoximine (BSO), makes these cells extremely sensitive to endogenous stress, unlike control cells that show moderate signs of alterations only at much higher BSO concentrations.

In addition, this cellular assay was used to identify several classes of compounds that are usefull for the treatment of Friedreich Ataxia. Surprisingly, it was observed that glutathione peroxidase (GPX) mimetics are also active in this assay, indicating that this class of compounds is suitable for treatment of Friedreich Ataxia.

Furthermore, the present invention demonstrates that FRDA fibroblasts are selenium starved in contrast to control cells and that selenium supplementation is a therapeutic approach for treatment. Pretreatment of the cells with selenium, an intrinsic component of glutathione peroxidases (GPX), rescues FRDA cells from BSO stress, indicating that FRDA fibroblasts have an impaired selenium metabolism and that their sensitivity towards oxidative stress is at least partly due to the fact that they have impaired GSH-dependent detoxification mechanisms.

Therefore in a second aspect, the present invention relates to the use of GPX mimetics, preferably Ebselen, for the preparation of a medicament for the treatment of Friedreichs Ataxia.

"GPX-mimetics" according to the invention are any compound that mimics to at least some extent the catalytic reactions performed by GPX in the presence of its substrates and cofactors. They do not have peptidomimetic structures, e.g. do not contain peptide bonds. In particular, small-molecule GPX mimetics are not proteinergic enzymes or peptide fragments thereof.

The following references are guidelines for the definition of GPX mimetics:
Aumann et al. "Glutathione peroxidase revisited. Simulation of the catalytic cycle by computer-assisted molecular modelling." Biomedical and Environmental Sciences 10, 1997, 136-155. Wilson et al. "Development of synthetic compounds with glutathione peroxidase activity". J. Am. Chem. Soc. 111, 1989, 5936-5939. lwaoka M., Tomoda S. "A model study on the effect of an amino group of the antioxidant activity of glutathione peroxidase". J. Am. Chem. Soc. 116, 1994, 2557-2561. Chaudiere J. et al. "Design of new selenium-containing mimics of glutathione peroxidases" in: Oxidative Processes and Antioxidants (Paoletti et al., eds), Raven Press, New York, 1994.Mugesh & du Mont. "Structure-activity correlation between natural glutathione peroxidase (GPX) and mimics: a biomimetic concept for the design and synthesis of more efficient GPX mimics". Chem. Eur. J., 7, 2001, 1365-1370.

Preferably, GPX mimetics mimic the active site of the enzyme, where two amino acids (glutamine-70 and tryptophan-148) are located in close proximity to the selenocysteine as the reactive center (catalytic triad). For example, Wilson et al (J. Am Chem. Soc. 111, pp5936-5939 (1989) Development of synthetic compounds with glutathione peroxidase activity) suggested guidelines for the construction of GPX mimetics, in particular they suggested the inclusion of a strong basic group in proximity to the active selenium atom. lwaoka M. and Tomoda S. (J. Am Chem. Soc. 116, pp2557-2561 (1994) A model study on the effect of an amino group on the antioxidant activity of glutathione peroxidase) also emphasized the importance of a nitrogen base proximal to the selenium atom, they also suggested a direct Se-N interaction in a reaction intermediate preventing further oxidation of the selenium moiety, also allowing the regeneration of the selenol intermediate. According to these authors GPX mimetics can nevertheless have catalytic cycles that can differ from the enzyme actual reaction cycle.

More preferably, small molecule GPX-mimetics are mono- or diseleno small molecule mimetics.

In a preferred embodiment, the present invention relates to the use of a diseleno compound of the general formula l, 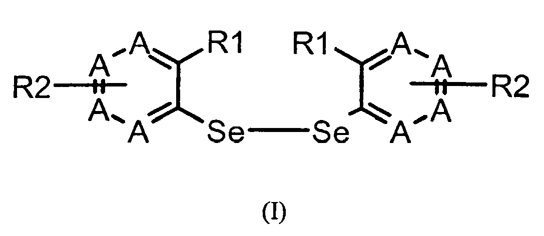 or formula II 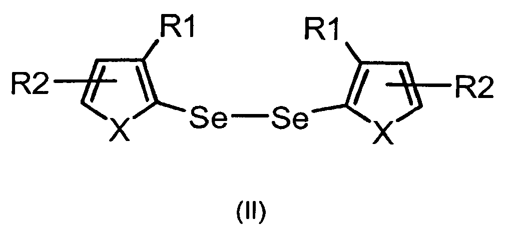 wherein
- A: denotes, in each case independently for each aromatic substituent, (a) C for all positions or (b) one N and C for all other positions of the aromatic substituent,
- X: denotes, in each case independently for each aromatic substituent, S, O, NH, NR₄, wherein R₄ denotes a linear or branched, saturated or unsaturated C₁₋₁₀ alkyl,
- R₁: denotes, in each case independently for each aromatic substituent, a hydrogen, primary or secondary, linear or branched, saturated or unsaturated C₁₋₆ alkohol, a primary or secondary, linear or branched, saturated or unsaturated C₁₋₆ ether, a primary, secondary or tertiary, linear or branched or cyclic saturated or unsaturated, C₁₋₈ amine, an alkyl substituted C₁₋₆ urea, or an alkyl and/or aryl substituted imidazoline,
- R₂: denotes, in each case independently for each selenium substituent, a hydrogen, a primary or secondary, linear or branched, saturated or unsaturated C₁₋₆ alkyl, a primary or secondary, linear or branched, saturated or unsaturated C₁₋₆ ether, or a nitro, trifluormethyl, sulfo or halo,
and its diastereomers or enantiomers and pharmaceutically acceptable salts thereof for the preparation of a medicament for the treatment of Friedreichs Ataxia.

In a further preferred embodiment, the present invention relates to the use of a seleno compound of the general formula III, 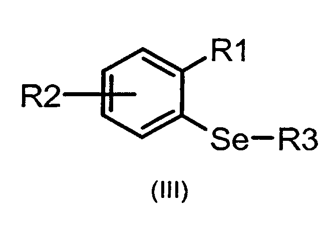 wherein
- R₁: denotes a primary or secondary, linear or branched, saturated or unsaturated C₁₋₆ alkohol, a primary or secondary, linear or branched, saturated or unsaturated C₁₋₆ ether, a primary, secondary or tertiary, linear or branched or cyclic saturated or unsaturated, C₁₋₈ amine, an alkyl substituted C₁₋₆ urea, or an alkyl and/or aryl substituted imidazoline,
- R₂: denotes a hydrogen, a primary or secondary, linear or branched, saturated or unsaturated C₁₋₆ alkyl, a primary or secondary, linear or branched, saturated or unsaturated C₁₋₆ ether or cyclic ether, or a nitro, sulfo or halo,
- R₃: denotes a primary or secondary, linear or branched, saturated or unsaturated, substituted or unsubstituted C₁₋₆ alcohol, non-cyclic or cyclic ether,
and its diastereomers or enantiomers and pharmaceutically acceptable salts thereof for the preparation of a medicament for the treatment of Friedreichs Ataxia.

R₁, as used in the general formulas I to III preferably denotes a secondary C₁₋₆ alkohol, a secondary C₁₋₆ ether, a secondary or tertiary, linear or cyclic C₁₋₈ amine, a 1,1-di-C₁₋₆ alkyl-3-C₁₋₆ alk-1-yl-urea, or a 1,3-di-C₁₋₆ alkyl-5-aryl imidazoline, more preferably a secondary C₁₋₄ alcohol, a secondary C₁₋₄ ether, a secondary or tertiary, linear or cyclic C₁₋₆ amine, or a 1,3-di- C₁₋₃ alkyl-5-aryl imidazoline, most preferably propan-2-ol, 1-hydroxypropyl, 1-ethoxyethyl, 1,3-Dimethyl-5-phenyl-imidazolidin-4-yl, 1-hydroxy-2,2-dimethyl-propyl, 1-hyroxy-butyl, 1-(dimethylamino)-ethyl, or 1-pyrrolidine-1-yl-eth-1-yl.

R₂, as used in the general formulas I to III preferably denotes a hydrogen, a primary or secondary, linear or branched, saturated or unsaturated C₁₋₄ alkyl, a primary or secondary, linear or branched, saturated or unsaturated C₁₋₄ ether, or a nitro, trifluoromethyl or halo, more preferably a hydrogen, a primary or secondary, linear or branched, saturated C₁₋₄ alkyl, a primary, linear, saturated C₁₋₄ ether, or a nitro, trifluoromethyl or halo, most preferably a tert-butyl, a methyl, a nitro, or a methoxy, a chloro, a bromo, a fluoro, or a trifluoromethyl.

R₃, as used in the general formula III preferably denotes a primary or secondary, linear or branched, saturated, substituted or unsubstituted C₁₋₃ alcohol, or non-cyclic C₁₋₃ ether, more preferably a phenyl-substituted primary or secondary saturated C₁₋₃ alcohol or C₁₋₃ ether, and most preferably a 2-hydroxy-1-phenyl-ethyl, a 2-methoxy-2-phenyl-ethyl.

R₄, as used in the general formula II preferably denotes a linear or branched, saturated or unsaturated C₁₋₄ alkyl, more preferably a linear or branched, saturated C₁₋₄ alkyl, and most preferably a methyl, ethyl, or isopropyl.

The aromatic substituent as used in the general formula I comprising A preferably is a phenyl or a 2-pyridil substituent.

X in the general formula II preferably denotes NH, or O, more preferably NH.

In a particularly preferred embodiment the present invention is directed to the use of a Bis[2-[1-(C₁₋₆ alkylamino)-C₁₋₆ alkyl]ferrocenyl]-diselenide compound for the preparation of a medicament for the treatment of Friedreichs Ataxia.

In a most preferred embodiment, the present invention relates to a GPX mimetic according to the invention, selected from the group consisting of Bis[2-(propan-2-ol)-phenyl]-diselenide, (S,S)-Bis[2-(1-hydroxypropyl)-5-tert-butyl-phenyl]-diselenide, (S,S)-Bis[3-(1-ethoxyethyl)-pyridine-2]diselenide, 1-[2-(2-Hydroxy-(S)-1 -phenyl ethyl selenyl)-phenyl]-propan-(R)-1-ol, 1-[2-(2-Hydroxy-(S)-1 -phenyl ethyl selenyl)-phenyl]-propan-(S)-1-ol, (S,S)-Bis[2-(1-hydroxypropyl)-6-methyl-phenyl]-diselenide, (S,S)-Bis[2-(1-hydroxypropyl)-4-nitro-phenyl]-diselenide, (S)-1-[3-Methoxy 2-(2-phenyl-tetrahydrofuran-3-yl-selenyl)-phenyl]-ethanol, Bis[2-(1,3-Dimethyl-(S)-5-phenyl-imidazolidin-(S)-4-yl)-phenyl]-diselenide, (Bis[2-(1-hydroxy-2,2-dimethyl-propylphenyl]-diselenide, Bis[4-methoxy-phenyl]-diselenide, (Bis[2-(1-hydroxy-butylphenyl]-diselenide, [R,S;R,S]-Bis[2-[1-(dimethylamino)-ethyl]ferrocenyl]-diselenide, (R,R)-Bis[2-(1,1-dimethyl-3-eth-1-yl-urea)-phenyl]-diselenide, (R,R)-Bis[2-(1-dimethylamino-eth-1-yl)-phenyl]-diselenide, (R,R)-Bis[2-(1-pyrrolidine-1-yl-eth-1 -yl)-phenyl]-diselenide for the preparation of a medicament for the treatment of Friedreichs Ataxia.

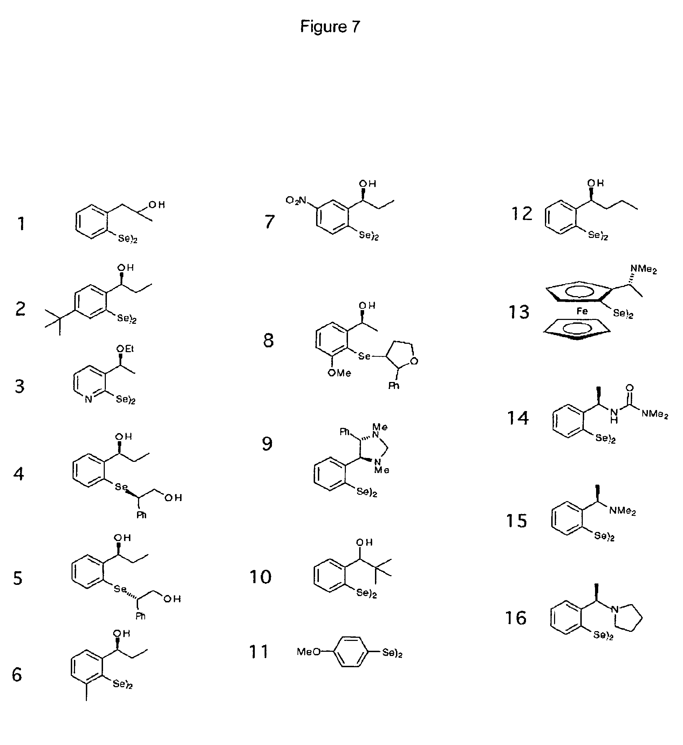

The above compounds are also described in figure 7.

In a preferred embodiment the compounds for use according to the invention are combined with free radical scavengers and/or antioxidants, preferably coenzyme Q10 or derivatives thereof, N-acetyl cysteine, and/or vitamin E or derivatives thereof.

While no therapy is known that can delay, stop, or reverse the progression of FRDA, free radical scavengers and antioxidants such as coenzyme Q10, N-acetyl cysteine, and vitamin E are currently being used for treatment trials.

In a preferred embodiment the compounds for use according to the invention are combined with compounds that are being evaluated for being useful for FRDA treatment such as buspirone, amantadine salts, Idebenone and/or neurotrophic factors, preferably insulin-like growth factor I (IGF-I) for the preparation of a medicament for the treatment of disorders caused by reduced Frataxin expression, preferably the treatment of Friedreichs Ataxia.

In a preferred embodiment the compounds for use according to the invention are combined with selenium.

In effecting treatment of a subject suffering from FRDA the compounds disclosed by the present invention for said purpose can be administered in any form or mode which makes the therapeutic compound bioavailable in an effective amount, including oral or parenteral routes. For example, products of the present invention can be administered intraperitoneally, intranasally, buccally, topically, orally, subcutaneously, intramuscularly, intravenously, transdermally, rectally, and the like. One skilled in the art in the field of preparing formulations can readily select the proper form and mode of administration depending upon the particular characteristics of the product selected, the disease or condition to be treated, the stage of the disease or condition, and other relevant circumstances. (Remington's Pharmaceutical Sciences, Mack Publishing Co. (1990)). The products of the present invention can be administered alone or in the form of a pharmaceutical preparation in combination with pharmaceutically acceptable carriers or excipients, the proportion and nature of which are determined by the solubility and chemical properties of the product selected, the chosen route of administration, and standard pharmaceutical practice. For oral application suitable preparations are in the form of tablets, pills, capsules, powders, lozenges, sachets, cachets, suspensions, emulsions, solutions, drops, juices, syrups, while for parenteral, topical and inhalative application suitable forms are solutions, suspensions, easily reconstitutable dry preparations as well as sprays. Compounds according to the invention in a sustained-release substance, in dissolved form or in a plaster, optionally with the addition of agents promoting penetration of the skin, are suitable percutaneous application preparations. The products of the present invention, while effective themselves, may be formulated and administered in the form of their pharmaceutically acceptable salts, such as acid addition salts or base addition salts, for purposes of stability, convenience of crystallization, increased solubility, and the like.
The amount of active agent to be administered to the patient depends on the patient's weight, on the type of application, symptoms and the severity of the illness. Normally 0.01 to 20 mg/kg of at least one substance of the general formula I to III, Ebselen and/or selenium are administered.
However, care must be exercised when administering selenium and selenium-containing compounds. Data from WHO sources, cited in Levander et al, Biomed Environm. Sci. 10, p214 (1997), mention a tentative poisoning threshold around 400 microgram selenium/day. Recommended daily allowance is up to 70 microgram selenium/day for an adult male. Due to its particular properties, selenium and selenium-containing compounds should be used according to the invention in non-toxic concentrations.

A third aspect of the present invention relates to the use of cells with reduced frataxin expression and a reduced cellular glutathione content for identifying and/or validating candidate substances for the treatment of Friedreich Ataxia (FRDA), preferably the use of cells derived or isolated from Friedreich Ataxia (FRDA)-patients with a reduced cellular glutathione content.

In a preferred embodiment an inhibitor of the γ-glutamyl cysteine synthase, preferably BSO (L-buthionine-(S,R)-sulfoxime), is added to said cells and said cells are cultured in selenium-restricted medium.

### Figures

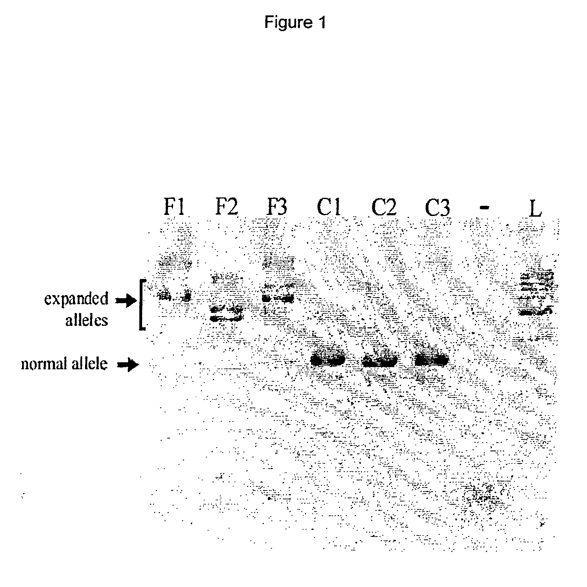

**Figure 1** relates to Example 1 and shows the result of the GAA genotyping of all 6 cell lines used. Agarose gel electrophoresis of PCR products encompassing the GAA repeat region of intron 1 of the Frataxin gene were analysed. C1-C3: control cell lines. F1-F3: FRDA cell lines. (-): no template control. L: size marker.
   With all control cell lines (C1-C3), a PCR product of 1.5kb was obtained, indicating no abnormal GAA extension. With all 3 FRDA cell lines (F1-F3), larger PCR products were obtained, in conjunction with the absence of the PCR product seen with normal cells. This indicates that all three FRDA cell lines had two expanded alleles at least 1200bp long (about 400 repeats).

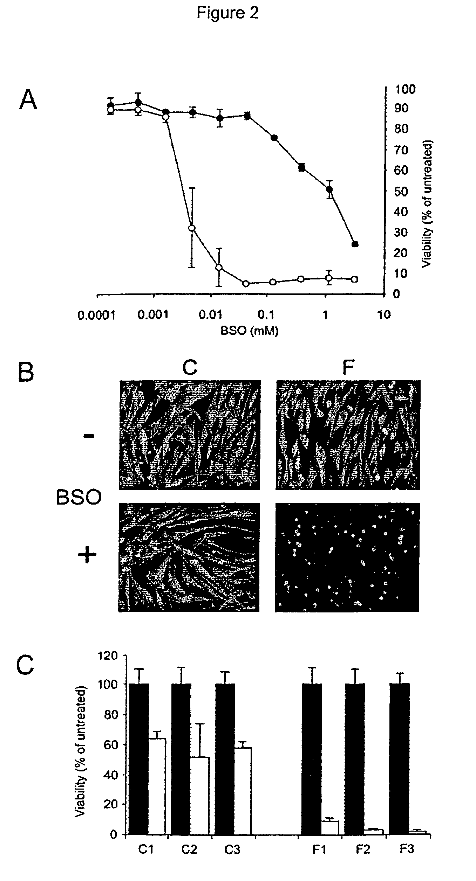

**Figures 2A, B and C** relate to example 2 and show:
   **A**: Dose-response curve for cell viability of BSO-treated control (filled circles) and FRDA fibroblasts (open circles). Data points indicate mean ± standard deviation, n=3
   **B**: Fluorescence microscopy analysis of control and FRDA fibroblasts stained with CalceinAM and ethidium homodimer: Upper panels (-): untreated cells, lower panels (+): BSO treated cells. Left panels (C): control fibroblasts, right panels (F): FRDA fibroblasts. Green color corresponds to calceinAM staining, whereas red color indicates ethidium homodimer staining (cell death).
   **C**: BSO effect (1 mM) on cell viability for different control (C1-C3) and FRDA (F1-F3) cell lines. Results (mean ± standard deviation expressed in per cent of untreated cells, *n*=4) are expressed as percent of the corresponding untreated cells.

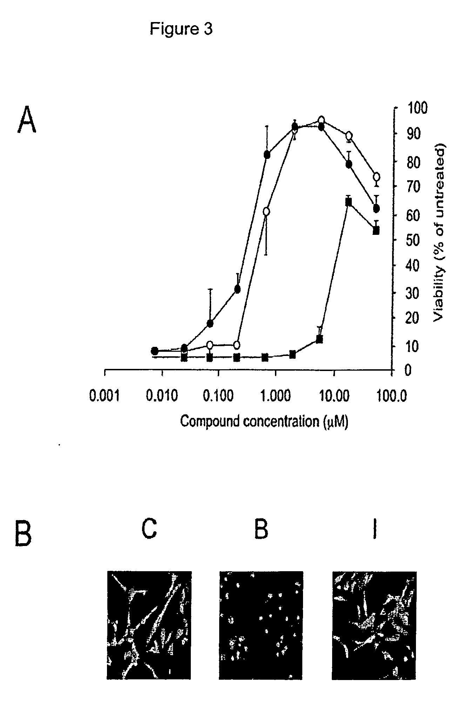

**Figures 3A and B** relate to example 4 and show a
   **A:** Dose response curves for cell survival upon BSO treatment. Results (mean ± standard deviation, *n*=4) are expressed as percent of the corresponding untreated cells. ldebenone (filled circles), Vitamin E (open circles), Trolox (filled squares).
   **B:** CalceinAM / Ethidium homodimer microscopy analysis of FRDA cells (as described in Figure 2): from left to right: untreated (C), 1 mM BSO treated (B), 1 mM BSO + 5µM Idebenone treated (I) FRDA cells. Green color indicates calceinAM staining, whereas red color indicates ethidium homodimer staining.

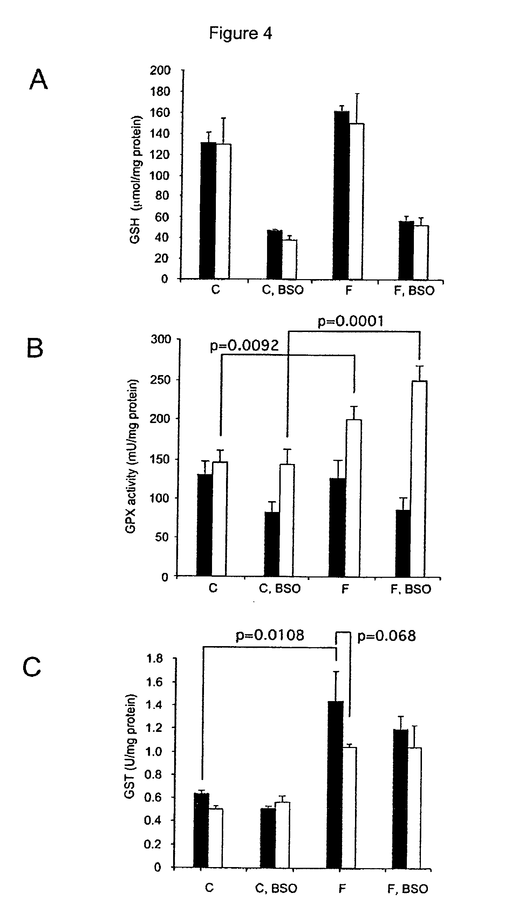

**Figures 4A, B and C** relate to Example 5 and shows the effect of BSO and selenium on intracellular glutathione, glutathione peroxidase activity and glutathione-S-transferases activities measurement:
   Black bars: no selenium supplementation, white bars: 500 nM sodium selenite. C: control cells, F: FRDA cells.
      **A:** GSH concentration, results are expressed as nmol GSH/mg protein ± standard deviation, (n=4).
      **B:** GPX enzymatic activities (maximum velocity of the NADPH consumption) are expressed as milliUnit enzyme/mg protein using tert-butyl hydroperoxide as substrate and purified bovine erythrocyte GPX as standard. Values are expressed as mean ± standard deviation, (*n*=4). To indicate significance values, *p*-values (student's unpaired t-test) are provided.
      **C:** GST activity are expressed as mean unit/mg protein ± standard deviation, (*n*=4). Horse glutathione-S-transferase was used as standard. To indicate significance values, *p*-values (student's unpaired t-test) are provided.

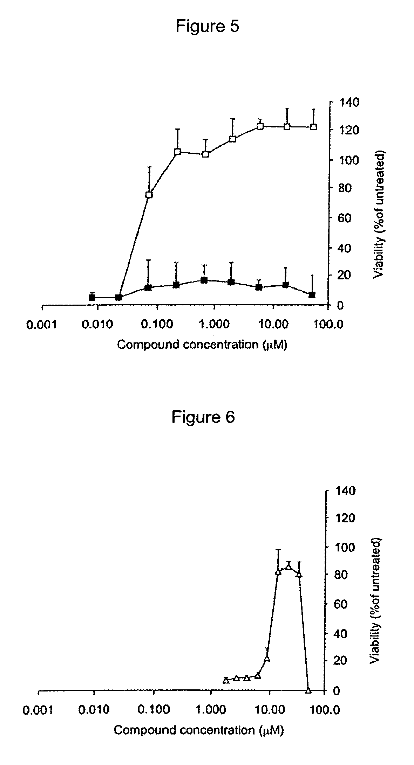

**Figure 5** relates to Example 6 and shows dose-response curves for cell viability following treatment with 1 mM BSO. For each data point, results (mean ± standard deviation, *n*=4 for each data point) are expressed as percent of untreated cells. open squares: 24 hours Na Selenite preincubation, filled squares: Na Selenite without preincubation.
**Figure 6** relates to Example 7 and shows dose response curves for cell viability following treatment with 1 mM BSO and increasing concentrations of Ebselen. Results (mean ± standard deviation, *n*=4 for each data point) are expressed as percent of untreated cells. For comparison, the scale of this figure is the same as in the previous figures.
**Figure 7** relates to Example 8 and shows the chemical structure of the small-molecule GPX mimetics tested.
   These compounds have been described in the following publications:
   Compound 1, 2, 3, 6, 7, 10 and 12: (Wirth, T. and G. Fragale, Asymmetric addition reactions with optimized selenium electrophiles, Chem. Eur. J., 3, 1894-1902, (1997)); compounds 4, 5 and 6: (Wirth, T., *et al.,* Mechanistic course of the asymmetric methoxyselenenylation reaction, J. Am. Chem. Soc., 120, 3376-3381, (1998)); compound 8: (Uehlin, L., et al., New and efficient chiral selenium electrophiles, Chem Eur. J., 8, 1125-1133, (2002)); compound 9: (Santi, C., *et al.,* Synthesis of a new chiral nitrogen containing diselenide as a precursor for selenium electrophiles, Tetrahedron: Asymmetry, 9, 3625-3628, (1998)); compound 11: (Wirth, T., Glutathione peroxidase-like activities of oxygen-containing diselenides, Molecules, 3, 164-166, (1998)); compound 13: (Nishibayashi, Y., *et al.,* Novel chiral ligands, diferrocenyl dichalcogenides and their derivatives, for rhodium- and iridium-catalyzed asymmetric hydrosilylation, Organometallics, 15, 370-379, (1996)); compounds 14, 15 and 16: (Wirth, T., *et al.,* Chiral diselenides from benzylamines. Catalysts in the diethylzinc addition to aldehydes, Helv. Chim. Acta, 79, 1957-1966, (1996)).

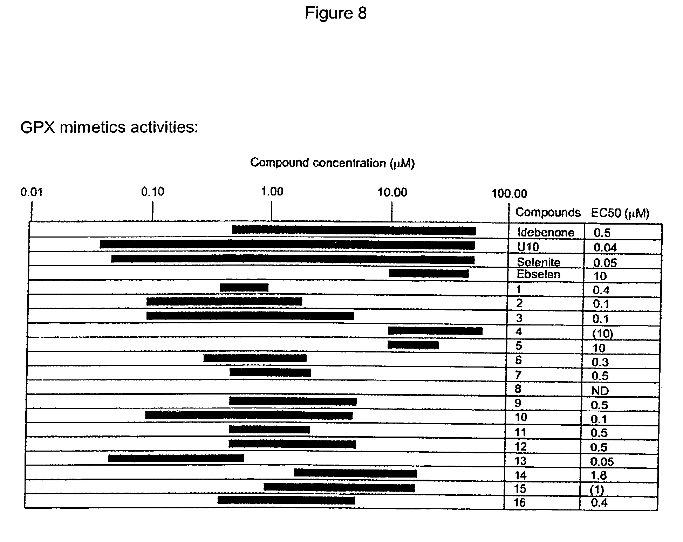

**Figure 8** relates to Example 8 and shows the working range of GPX mimetics tested.
   For each compound, a dose-response curve for survival of FRDA cells upon BSO treatment was generated. From these curves, the concentration range for which a specific compound produced at least 50% cell viability was measured and is indicated by black bars. The left hand side of the bars corresponds to EC50 values (also indicated in the right column). ND: not determined, compound did not reach the 50% cell viability threshold, but was active at the highest concentration tested (50 µM). U10 is Decylubiquinone (2,3-Dimethoxy-5-methyl-6-decyl-1,4-benzoquinone). Numbers under parenthesis: EC50 concentrations for compounds that did not produce 50% viability for none of the concentrations tested.

### Examples

The following examples further illustrate the best mode contemplated by the inventors of carrying out their invention.

### Example 1: Description of cell lines and culture conditions

Friedreich Ataxia primary culture fibroblast cell lines F1 and control fibroblast line C1 were provided by the Insel-Spital Bern (Switzerland), FRDA line F3 was provided by Hopital Necker, Paris (France), FRDA lines F2 and control lines C2 and C3 were obtained from Coriell Cell Repositories (Camden, NJ; catalog numbers GM04078, GM08402 and GM08399 respectively). Cells were grown in a humidified atmosphere supplemented with 5% CO₂. For experiments, the cells were trypsinized and resuspended at a density of 40000 cells/ml in growth medium consisting of 25% (v/v) M199 EBS and 64% (v/v) MEM EBS without phenol red (Bioconcept, Allschwil, Switzerland) supplemented with 10% (v/v) fetal calf serum (PAA Laboratories, Linz, Austria), 100 U/ml penicillin, 100 µg/ml streptomycin (PAA Laboratories, Linz, Austria), 10 µg/ml insulin (Sigma, Buchs, Switzerland), 10 ng/ml EGF (Sigma, Buchs, Switzerland) and 10 ng/ml bFGF (PreproTech, Rocky Hill, NJ) and 2 mM glutamine (Sigma, Buchs, Switzerland).
Molecular diagnosis of the cell lines were performed with a PCR-based approach. Cells grown to confluence in 10 cm dishes were used for DNA extraction using a DNA isolation kit (DNeasy, Qiagen, Hilden, Germany) according to the instructions of the manufacturer. PCR amplification of GAA repeats was performed with the primers "Bam" and "2500F" and the cycling protocol described in the literature (Campuzano, V., *et al.,* Friedreich's ataxia: autosomal recessive disease caused by an intronic GAA triplet repeat expansion, Science, 271, 1423-7, (1996)). The PCR reaction was performed using the Expand Long Template PCR kit (Roche Molecular Biochemicals, Mannheim, Germany) using a Biometra TGradient thermocycler (Biometra, Göttingen, Germany). PCR products were stained with SYBR Green (Molecular Probes, Eugene, OR) and separated by agarose gel electrophoresis.
For results, see figures 1.

### Example 2: Differential effect of BSO treatment on Friedreich Ataxia and control fibroblasts

This example shows the high sensitivity of FRDA fibroblasts towards oxidative stress generated by culture in the presence of BSO. This effect is specific for all FRDA cells tested. All FRDA cells die in the presence of BSO whereas control cell survive this treatment.
The cells were seeded in 96-well plates at a density of 4000 cells/well. They were incubated in the presence of various concentrations of L-buthionine-(S,R)-sulfoximine (BSO), an inhibitor of γ-glutamyl cysteine synthase (EC: 6.3.2.2), the rate limiting enzyme in the biosynthesis of glutathione. Cell viability was measured when the first signs of toxicity appeared in the controls (typically after 12 to 48 h). The cells were stained for 60 min at room temperature in PBS with 1.2 µM CalceinAM and 4 µM Ethidium homodimer (Live/Dead assay, Molecular Probes, Eugene, OR). The plates were washed twice with PBS and fluorescence intensity was measured with a Gemini Spectramax XS spectrofluorimeter (Molecular Devices, Sunnyvale, CA) using excitation and emission wavelengths of 485 nm and 525 nm respectively. Live cells imaging was performed with a Zeiss Axiovert 135M fluorescence microscope equipped with a cooled CCD camera (Sensicam, PCO Computer Optics, Kelheim, FRG). Image acquisition was performed with the ImagePro Plus software (Media Cybermetics, Silver Spring, Maryland).

Cell titer measurements after overnight exposure to BSO showed a slight decrease in control fibroblasts density at a BSO concentration of 100 µM and increasing reduction in cell titer in a dose-dependent fashion (Fig. 2, panel A). In contrast, FRDA cells showed a marked reduction in cell titer at a concentration as low as 4 µM BSO. Higher BSO concentrations decreased cell density further. This differential sensitivity to BSO can be observed over a broad concentration range and choose for further experiments a discriminating BSO concentration of 1 mM were chosen. Staining of unfixed cells with calceinAM and ethidium homodimer to reveal live and dead cells respectively showed that only BSO-treated FRDA cells had ethidium homodimer permeable plasma membranes, indicating that the BSO treatment caused cell death. (Fig. 2, panel B). Untreated cells and BSO-treated control fibroblasts were stained in a similar fashion with calceinAM and produced no red fluorescence with ethidium homodimer staining, indicating that they survived the BSO treatment.
This FRDA-specific BSO sensitivity was verified with several primary cell lines obtained from control donors and from FRDA patients. All control fibroblasts displayed moderate sensitivity to BSO (average 60% viability at 1 mM BSO) compared to the FRDA cells that had less than 10% viability compared to their respective untreated controls (Fig. 2, panel C). A 2-way ANOVA revealed a significant effect of cell type (F=1391.1, DF=5, p<0.001), BSO (F=1538.8, DF=1, p<0.001) and the interaction between the two (F=100.2, DF=5, p<0.001).

### Example 3: ldebenone can rescue FRDA cells from cell death induced by BSO treatment

FRDA cells were preincubated for 24 hours with Idebenone, Vitamin E or Trolox and were then subjected to 1 mM BSO treatment. Cell viability was measured as described in Example 2.
By applying various concentrations of ldebenone (2,3-dimethoxy-5-methyl-6-(10-hydroxydecyl)-1,4-benzoquinone) to the cells prior to the BSO treatment, it was observed that this molecule could prevent cell death, with an EC50 value of 0.5 µM (Fig. 3 panel A). Full rescue was observed at 2 µM and at the highest concentration tested (50 µM), the rescue effect was slightly diminished, probably because of the pro-oxidant properties of Idebenone at these concentrations (Semsei, I., *et al.,* In vitro studies on the OH. and O2.- free radical scavenger properties of idebenone in chemical systems, Arch. Gerontol. Geriatr, 11, 187-197, (1990)) ). Upon microscopic examination, the Idebenone-treated cells appeared undistinguishable from untreated cells (Fig. 3 panel B). This result surprisingly demonstrates that this cell-based screening system can detect Idebenone, currently the only drug being tested successfully for FRDA patient treatment (Schulz, et al., "Oxidative stress in patients with Friedreich ataxia", Neurology, 55, 1719-21., (2000) ; Rustin, et al., "Effect of idebenone on cardiomyopathy in Friedreich's ataxia: a preliminary study", Lancet, 354, 477-9., (1999) ; Lerman-Sagie, et al., "Dramatic improvement in mitochondrial cardiomyopathy following treatment with idebenone", J. Inherit Metab Dis, 24, 28-34., (2001). Hausse, et al., "Idebenone and reduced cardiac hypertrophy in Friedreich's Ataxia" Heart, 87: 346-349 (2002)).

It was verified whether known antioxidant molecules could rescue FRDA cells death in this assay. Vitamin E was almost as potent as Idebenone in preventing cell death, with an EC50 value of 0.7 µM (Fig. 3 panel A). Surprisingly, Trolox (6-hydroxy-2,5,7,8-tetramethylchroman-2-carboxylic acid), a water soluble derivative of vitamin E, lacking the long carbon side chain, was much less efficient in preventing cell death. It preserved only 60% cell viability at the optimal concentration of 25 µM. At higher concentrations, activity dropped to 55% cell viability. It was concluded from these data that the cell rescue effect observed here is not a general antioxidant property, but that this effect is rather specific to the stress generated in this assay and/or to the compound's lipophilicity.

### Example 4: Effect of CoenzymeQ variants

This example shows that various chain length CoenzymeQ variants are potent in rescuing FRDA cells death upon BSO treatment. However, a certain lipophilic property seems to be required for activity.
FRDA cells were preincubated for 24 hours with various concentrations of the indicated compounds and then subjected to 1 mM BSO stress. Cell viability was measured after a further 48 hours incubation as described in Example 2. EC50 values were deduced from the dose-response curves. The natural CoQ10 was not active in this assay (Table 1). However, a possible bioavailability problem with this molecule due to its 50 carbon atom side chain can not be ruled out. CoQ1 had EC50 values similar to Idebenone (0.2 µM). CoQ2 performed better than Idebenone, with an EC50 value of 30 nM. CoQ0 was inactive, indicating that a certain lipophilicity is necessary. Decylubiquinone was almost as effective as CoQ2, with an EC50 of 40 nM.

**Table 1**

| | | | |
|---|---|---|---|
| | | Carbon side chain length | EC 50 (µM) |
| 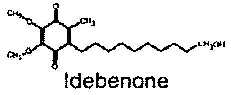 | | 10 | 0.5 |
| 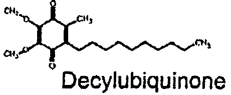 | | 10 | 0.04 |
| 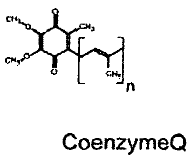 | CoQ0 n=0 | 0 | n.a. |
| | CoQ1 n=1 | 5 | 0.2 |
| | CoQ2 n=2 | 10 | 0.03 |
| | CoQ10 n=10 | 50 | >50 |

Table 1: The chemical structure of the various Idebenone analogs are indicated, as well as the length of their carbon chain tail (in carbon atoms). For CoenzymeQ variants, the number of isoprene units (n) is indicated. CoQ10 did not reach the 50% cell viability rescue at the highest dose tested. The short chain CoQ0 was not active in this test (n.a.).

### Example 5: Glutathione metabolism in FRDA cells:

This example shows that FRDA cells do not have an altered intracellular glutathione (GSH) concentration. Surprisingly however, the FRDA cells have an altered glutathione peroxidase response to selenium supplementation and have higher glutathione-S-transferases activity than control cells.
Control and FRDA cells (5X10⁵ cells grown in 10cm dishes) were preincubated with sodium selenite (500 nM) for 24 hours and then subjected to 1 mM BSO treatment for 24 hours. Total cell extracts were used to measure glutathione content, glutathione peroxidase activity and glutathione-S-transferase activity.
To determine the glutathione content, cells were trypsinized, washed twice with PBS and snap frozen in 100 µl PBS. Cells were lysed in PBS supplemented with a protease inhibitor cocktail (Complete, Roche Diagnostics, Rotkreuz, Switzerland) by 4 freeze/thaw cycles. Total protein content was measured with the BioRad protein assay (BioRad, Hercules, CA) using calibrated bovine serum albumin solutions as standards (Pierce). Reduced glutathione content was measured essentially as described (Kamencic, H., *et al*., Monochlorobimane fluorometric method to measure tissue glutathione, Anal Biochem, 286, 35-7., (2000)) with a final monochlorobimane (mCIB) (Molecular Probes, Eugene, OR) concentration of 25 µM. The GSH-mCIB adduct fluorescence was measured with a Gemini spectrofluorimeter using excitation and emission wavelengths of 380 nm and 470 nm respectively. Known amounts of reduced glutathione were used as standards.
To determine the glutathione peroxidase activity, cell extracts obtained for total GSH measurements were adjusted with PBS to a final protein concentration of 1.85 mg/ml. Enzymatic activity was measured with the Glutathione peroxidase cellular activity assay kit (Sigma, St Louis, MO) essentially according to the instructions of the manufacturer: Enzymatic activity was measured with 40 µg total protein extract in a final reaction volume of 100 µl. NADPH consumption was measured by the decrease in NADPH fluorescence at 445 nm. All measurements were done in triplicate in 96-well plates with a Gemini spectrofluorimeter using excitation and emission wavelengths of 340 nm and 445 nm respectively.
Glutathione-S-transferase activity was determined in the same way as the glutathione content, except that the assay was performed in the presence of an excess of glutathione (1 mM) instead of an excess of exogenous glutathione-S-transferase.

Because the BSO treatment is known to deplete cellular (GSH) levels, intracellular GSH concentrations were measured first. Any difference in basal glutathione levels between the two cell lines that were tested were not observed (Fig. 4, panel A). As expected, BSO treatment lead to a reduction in glutathione levels in both cell types. Preincubation of the cells with selenium did not prevent the glutathione depletion. (Fig. 4, panel A). From this, it was concluded that the GSH levels *per se* are not responsible for the observed difference in phenotype between the two cell types.
Because GSH does not seem to be responsible for the observed difference between FRDA and control cells upon BSO treatment, the high sensitivity of FRDA cells towards BSO could be due to a lower GPX activity that would rapidly become rate limiting in GSH-depletion conditions. Therefore, GPX enzymatic activity in extracts from both cell types was compared. This measurement is based on the recycling of oxidized glutathione generated by GPX, using tert-butyl hydroperoxide as substrate. Oxidized glutathione is reduced by an excess exogenous glutathione reductase that will consume a corresponding amount of NADPH that is then measured by fluorescence. There is no difference in basal GPX activity between the control and the FRDA cells (Fig. 4, panel B). The BSO treatment produced a small decrease in enzyme activity, in both cell types.
GPX are selenoproteins, the selenium atom beeing an integral part of the enzyme active site. GXP activity is strongly dependent on the selenium status of the cell. Therefore, both cell types were supplemented with selenium and GPX activity measured. Both cell types responded differently to selenium supplementation. Whereas in control cells the basal GPX activity was not enhanced by selenium, in FRDA cell a 50% increase in enzyme activity was observed. Moreover, in control cells, selenium supplementation prevented the enzyme activity loss due to BSO treatment. In the case of FRDA cells, not only did selenium improve basal GPX activity by 50%, but in contrast to control cells, selenium increased GPX activity by a factor of 3 despite the BSO treatment, reaching levels twice as high as the basal levels found in control cells.
These data show that control cells do not benefit from selenium supplementation in the absence of BSO treatment. Selenium does not lead to an upregulation of enzymatic activity, therefore these cells can be considered selenium-replete, as far as GPX activity is concerned. On the other hand, although FRDA cells have basal GPX activity levels similar to control cells, selenium supplementation leads to an upregulation of the enzyme activity. It is hypothesised that these cells have an increased basal demand for GPX activity, but due to an impairment in selenium metabolism, they are unable to produce more active GPX unless they are supplemented with selenium, the latter being rate limiting for GPX synthesis. In the presence of BSO, these cells could respond to the increased oxidative stress by upregulating their GPX levels, however, this is only possible in the presence of higher-than-normal selenium levels. In these conditions (higher selenium) the FRDA cells survive the BSO stress. From this it is concluded that the protective effect of selenium in FRDA cells results from an enhanced production of active GPX, as monitored by elevated GPX activity in this experiment, that allows the cells to control the additional oxidative stress generated by BSO.

In order to substantiate this hypothesis, glutathione-S-transferase (GST) activities in the two cell types were measured. To a certain extent, GST are capable of performing detoxification reactions similar to GPX and several reports indicate that GST can be upregulated in selenium deficiency (Arthur, J. R., *et al.,* "The effect of selenium and copper deficiencies on glutathione S-transferase and glutathione peroxidase in rat liver", Biochem J, 248, 539-544, (1987); Beckett, G. J., *et al.,* "The changes in hepatic enzyme expression caused by selenium deficiency and hypothyroidism in rats are produced by independent mechanisms", Biochem J, 266, 743-7., (1990) ; Mehlert, A. and A. T. Diplock, "The glutathione S-transferases in selenium and vitamin E deficiency", Biochem J, 227, 823-831, (1985)). However, GST are not selenoproteins, and in contrast to GPX, they conjugate GSH to the toxic compound rather than using GSH reducing power. So these enzymes consume GSH.
By comparing glutathione-S-transferase activities in diseased and normal cells, it was found that FRDA cells have more than twice as much GST activity than control cells (Fig. 4, panel C). In control cells, the GST activity is neither modulated by Selenium, nor by BSO. In contrast in FRDA cells, Selenium treatment slightly reduces the GST activity (with a parallel increase in GPX activity, as shown in Fig. 4, panel B).

Taken together, these data indicate that FRDA cells have an impaired selenium metabolism, as shown by their response to selenium supplementation. As expected from results from animal studies, a selenium deficiency leads to an increased GST activity, as a backup detoxification mechanism.

### Example 6: Effect of sodium selenite and BSO on FRDA fibroblasts

This example shows that FRDA cells are sensitive to BSO because of an alteration of their selenium metabolism.
FRDA cells were incubated with increasing concentrations of sodium selenite (prepared as a stock solution 5 mM in PBS) for 24 hours, then subjected to BSO stress. Cell viability was measured as described in Example 2.
In the absence of selenium supplementation, FRDA cells are vulnerable to BSO treatment (Example 2). Addition of 200 nM sodium selenite to the cells protected them fully, and higher concentrations even enhanced cell growth (Fig. 5). When added at the same time as BSO, selenium did not protect the cells. This suggests that the selenium effect, described also in Example 5, could be mediated through a time-dependent process like *de novo* protein synthesis.

### Example 7: 2-phenyl-1,2-benzisoselenazol-3-(2H)-one (Ebselen) protects FRDA fibroblasts from BSO-mediated stress

Based on the previous results, the hypothesis whether small-molecules GPX mimetics could rescue the cell death phenotype of FRDA cells when exposed to BSO was tested. FRDA cells were incubated for 24 hours in the presence of Ebselen, a GPX mimetic (Muller, A., *et al.,* "A novel biologically active seleno-organic compound--I. Glutathione peroxidase-like activity in vitro and antioxidant capacity of PZ 51 (Ebselen)", Biochem Pharmacol, 33, 3235-9., (1984)) and then subjected to 1 mM BSO treatment. Surprisingly, it was found that Ebselen, in a narrow concentration range (between 10 and 50 µM) was able to rescue the cell death of FRDA cells (Fig. 6).

### Example 8: Small molecule GPX mimetics can rescue FRDA cells from BSO-mediated stress

This example shows that other small molecule glutathione peroxidase mimetics are active against the BSO induced stress in FRDA cells.
FRDA cells were incubated in the presence of a number of GPX mimetics. Two classes of molecules (Fig. 7) were evaluated: monoselenides (compound number 4, 5 and 8) and diselenides (compound number 1-3, 6, 7, 9-16). Dose-response curves were obtained for each molecule. From the dose-response curves not only the EC50, but also the concentration range for which a molecule produced at least 50% cell survival upon BSO challenge was calculated (Fig. 8).
GPX mimetics were tested as described in Example 3. Dose-response curves were obtained for all compounds tested. The respective concentration range for which a molecule produced at least 50% cell viability (compared to untreated controls) is indicated by a bar. The left hand side of these bars represent EC50 values.
Surprisingly, all GPX mimetics tested were effectively rescuing cell viability from the BSO effect. In general, all small molecule GPX mimetics had a narrow working range, (typically 1.5 orders of magnitude) above which most compounds had a toxic effect. Diselenides were in general more potent than monoselenides. On the contrary, antioxidants had a much wider working range, up to 3 orders of magnitude for decylubiquinone.

## Claims

1. Method for identifying and/or validating candidate substances for the treatment of Friedreich Ataxia, comprising the steps of:
a) providing cells with reduced frataxin expression,
b) incubating cells of step a) in selenium-restricted medium,
c) reducing the cellular gluthathione content of the cells in b),
d) contacting the cells of step c) with a candidate substance, and
e) evaluating the response of the cells of step d),
wherein steps b), c) and d) may also be performed in any other order than b), c) and d), the order b), c) and d) being preferred.

2. Method according to claim 1, **characterized in that** the cells of step a) are cells isolated or derived from Friedreich Ataxia (FRDA)-patients, preferably fibroblast cells derived from Friedreich Ataxia (FRDA)-patients.

3. Method according to claim 1 or 2, **characterized in that** in step c) the cellular glutathione content is reduced by inhibiting the *de novo* synthesis of glutathione.

4. Method according to claim 3, **characterized in that** the cellular glutathione content is reduced by the addition of an inhibitor of the γ-glutamyl cysteine synthetase, preferably BSO (L-buthionine-(S,R)-sulfoximine).

5. Method according to any one of claims 1 to 4, **characterized in that** said response in step e) is increased plasma membrane permeability and/or cell death.

6. Method according to any one of claims 1 to 5, **characterized in that** said response in step e) is compared to the response of control cells with normal frataxin expression and/or normal cellular glutathione content and/or under selenium-supplemented incubation conditions to said candidate substance.

7. Method according to any one of claims 1 to 6, **characterized in that** said response in step e) is compared to the response of control cells with reduced frataxin expression and reduced cellular glutathione content grown in selenium-restricted medium to a known effective candidate substance, preferably compared to the response of FRDA-fibroblasts, which are reduced in cellular glutathione content, to Idebenone (6-(10-hydroxydecyl)-2,3-dimethoxy-5-methyl-1,4-benzoquinone), or Ebselen (2-phenyl-1,2-benziso-selenazol-3-(2H)-one).

8. Use of a GPX mimetic for the preparation of a medicament for the treatment of Friedreich Ataxia.

9. Use according to claim 8, **characterized in that** the GPX mimetic is Ebselen (2-phenyl-1,2-benzisose(enazol-3-(2H)-one).

10. Use of GPX mimetics or GPX mimetics in combination with Idebenone (6-(10-hydroxydecyl)-2,3-dimethoxy-5-methyl-1,4-benzoquinone) for the preparation of a medicament for the treatment of Friedreich Ataxia.

11. Use according to claim 8 or 10, wherein small molecule GPX-mimetics, preferably mono- or diseleno small molecule mimetics are used.

12. Use according to claim 11, wherein a diseleno compound of the general formula I, 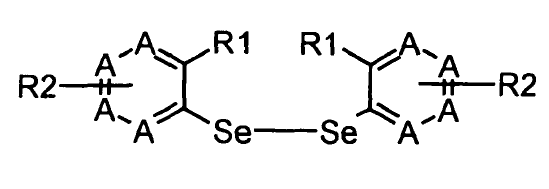 or formula II 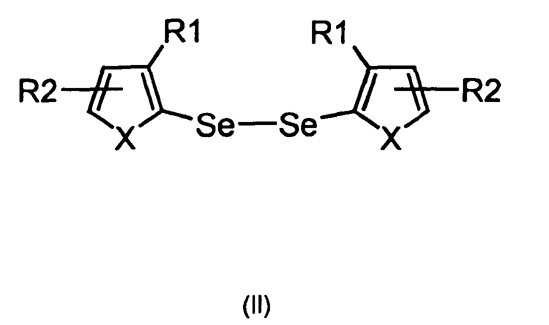 is used, wherein
A denotes, in each case independently for each aromatic substituent, (a) C for all positions or (b) one N and C for all other positions of the aromatic substituent,
X denotes, in each case independently for each aromatic substituent, S, O, NH, NR₄, wherein R₄ denotes a linear or branched, saturated or unsaturated C₁₋₁₀ alkyl,
R₁ denotes, in each case independently for each aromatic substituent, a hydrogen, primary or secondary, linear or branched, saturated or unsaturated C₁₋₆ alkohol, a primary or secondary, linear or branched, saturated or unsaturated C₁₋₆ether, a primary, secondary or tertiary, linear or branched or cyclic, saturated or unsaturated, C₁₋₈ amine, an alkyl substituted C₁₋₆ urea, or an alkyl and/or aryl substituted imidazoline,
R₂ denotes, in each case independently for each aromatic substituent, a hydrogen, a primary or secondary, linear or branched, saturated or unsaturated C₁₋₆ alkyl, a primary or secondary, linear or branched, saturated or unsaturated C₁₋₆ ether, or a nitro, trifluoromethyl, sulfo or halo,
and its diastereomers or enantiomers and pharmaceutically acceptable salts thereof.

13. Use according to claim 11, wherein the monoseleno compound has the general formula III, 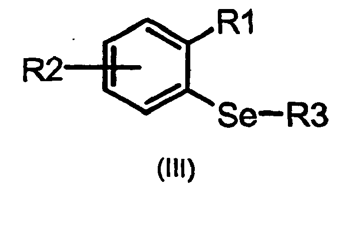 wherein
R₁ denotes a primary or secondary, linear or branched, saturated or unsaturated C₁₋₆ alkohol, a primary or secondary, linear or branched, saturated or unsaturated C₁₋₆ ether, a primary, secondary or tertiary, linear or branched or cyclic, saturated or unsaturated, C₁₋₈ amine, an alkyl substituted C₁₋₆ urea, or an alkyl and/or aryl substituted imidazoline.
R₂ denotes a hydrogen, a primary or secondary, linear or branched, saturated or unsaturated C₁₋₆ alkyl, a primary or secondary, linear or branched, saturated or unsaturated C₁₋₆ ether or cyclic ether, or a nitro, sulfo, trifluoromethyl or halo,
R₃ denotes a primary or secondary, linear or branched, saturated or unsaturated, substituted or unsubstituted C₁₋₆ alcohol, non-cyclic or cyclic ether,
and its diastereomers or enantiomers and pharmaceutically acceptable salts thereof.

14. Use of a seleno compound according to claim 12 or 13, wherein R₁ denotes a secondary C₁₋₆ alkohol, a secondary C₁₋₆ ether, a secondary or tertiary, linear or cyclic C₁₋₈ amine, a 1,1-di-C₁₋₆ alkyl-3-C₁₋₆ alk-1-yl-urea, or a 1,3-di-C₁₋₆ alkyl-5-aryl imidazoline, preferably a secondary C₁₋₄ alcohol, a secondary C₁₋₄ ether, a secondary or tertiary, linear or cyclic C₁₋₆ amine, or a 1,3-di- C₁₋₃ alkyl-5-aryl imidazoline, more preferably propan-2-ol, 1-hydroxypropyl, 1-ethoxyethyl, 1,3-Dimethyl-5-phenyl-imidazolidin-4-yl, 1-hydroxy-2,2-dimethyl-propyl, 1-hyroxy-butyl, 1-(dimethylamino)-ethyl, or 1-pyrrolidine-1-yl-eth-1-yl.

15. Use of a seleno compound according to any one of claims 12 to 14, wherein R₂ denotes hydrogen, a primary or secondary, linear or branched, saturated or unsaturated C₁₋₄ alkyl, a primary or secondary, linear or branched, saturated or unsaturated C₁₋₄ ether, or a nitro, trifluoromethyl or halo, preferably a hydrogen, a primary or secondary, linear or branched, saturated C₁₋₄ alkyl, a primary, linear, saturated C₁₋₄ ether, or a nitro, trifluoromethyl or halo, more preferably a tert-butyl, a methyl, a nitro, or a methoxy, a chloro, a bromo, a fluoro, or a trifluoromethyl.

16. Use of a seleno compound according to claim 13 to 15, wherein R₃ denotes a primary or secondary, linear or branched, saturated, substituted or unsubstituted C₁₋₃ alcohol, or non-cyclic C₁₋₃ ether, preferably a phenyl-substituted primary or secondary saturated C₁₋₃ alcohol or C₁₋₃ ether, and more preferably a 2-hydroxy-1-phenyl-ethyl, a 2-methoxy-2-phenyl-ethyl.

17. Use of a seleno compound according to claim 12, wherein R₄ denotes a linear or branched, saturated or unsaturated C₁₋₄ alkyl, preferably a linear or branched, saturated C₁₋₄ alkyl, and more preferably a methyl, ethyl, or isopropyl.

18. Use of a seleno compound according to claims 12, 14 or 15, wherein the aromatic substituent comprising A is a phenyl or a 2-pyridil substituent.

19. Use of a seleno compound according to claims 12, 14 or 15, wherein X denotes NH or O, preferably NH.

20. Use of a Bis[2-[1-(C₁₋₆ alkylamino)-C₁₋₆ alkyl]ferrocenyl]-diselenide compound for the preparation of a medicament for the treatment of Friedreichs Ataxia.

21. Use of a GPX mimetics according to any one of claims 8, and 10 to 20, wherein the mimetic is selected from Bis[2-(propan-2-ol)-phenyl]-diselenide, (S,S)-Bis[2-(1-hydroxypropyl)-5-tert-butyl-phenyl]-diselenide, (S,S)-Bis[3-(1-ethoxyethyl)-pyridine-2]diselenide, 1-[2-(2-Hydroxy-(S)-1-phenyl ethyl selenyl)-phenyl]-propan-(R)-1-ol, 1-[2-(2-Hydroxy-(S)-1-phenyl ethyl selenyl)-phenyl]-propan-(S)-1-ol, (S,S)-Bis[2-(1-hydroxypropyl)-6-methyl-phenyl]-diselenide, (S,S)-Bis[2-(1-hydroxypropyl)-4-nitro-phenyl]-diselenide, (S)-1-[3-Methoxy 2-(2-phenyl-tetrahydrofuran-3-yl-selenyl)-phenyl]-ethanol, Bis[2-(1,3-Dimethyl-(S)-5-phenyl-imidazolidin-(S)-4-yl)-phenyl]-diselenide, (Bis[2-(1-hydroxy-2,2-dimethyl-propylphenyl]-diselenide, Bis[4-methoxy-phenyl]-diselenide, (Bis[2-(1 -hydroxy-butylphenyl]-diselenide, [R,S;R,S]-Bis[2-[1-(dimethylamino)-ethyl]ferrocenyl]-diselenide, (R,R)-Bis[2-(1,1-dimethyl-3-eth-1-yl-urea)-phenyl]-diselenide, (R,R)-Bis[2-(1-dimethylamino-eth-1-yl)-phenyl]-diselenide, (R,R)-Bis[2-(1-pyrrolidine-1-yl-eth-1-yl)-phenyl]-diselenide.

22. Use according to any one of claims 8 to 21, wherein the seleno compound is combined with free radical scavengers and/or antioxidants, preferably coenzyme Q10 or derivatives thereof, N-acetyl cysteine, and/or vitamin E or derivatives thereof.

23. Use according to any one of claims 8 to 22 in combination with buspirone, amantadine salts, Idebenone and/or neurotrophic factors, preferably insulin-like growth factor I (IGF-l).

24. Use according to any one of claims 11 to 23 in combination with selenium.

25. Use of cells with reduced frataxin expression and a reduced cellular glutathione content for identifying and/or validating candidate substances for the treatment of Friedreich Ataxia (FRDA), preferably cells with a reduced cellular glutathione content derived or isolated from Friedreich Ataxia (FRDA)-patients.

26. Use according to claim 25, **characterized in that** an inhibitor of the γ-glutamyl cysteine synthetase, preferably BSO (L-buthionine-(S,R)-sulfoximine), is added to said cells and said cells are cultured in selenium-restricted medium.

## Patentansprüche

1. Verfahren zum Identifizieren und/oder Validieren von Kandidatensubstanzen zur Behandlung der Friedreich-Ataxie, umfassend die Schritte:
a) Bereitstellen von Zellen mit reduzierter Frataxin-Expression,
b) Inkubieren von Zellen aus Schritt a) in Selen-beschränktem Medium,
c) Reduzieren des zellulären Glutathiongehalts der Zellen in b),
d) In Kontakt Bringen der Zellen aus Schritt c) mit einer Kandidatensubstanz und
e) Evaluieren der Reaktion der Zellen aus Schritt d),
wobei die Schritte b), c) und d) auch in jeder anderen Reihenfolge als b), c) und d) durchgeführt werden können, wobei die Reihenfolge b), c) und d) bevorzugt wird.

2. Verfahren gemäß Anspruch 1, **dadurch gekennzeichnet, dass** es sich bei den Zellen aus Schritt a) um Zellen handelt, die von Patienten mit Friedrich-Ataxie (FRDA) isoliert oder abgeleitet wurden, vorzugsweise um Fibroblastenzellen, die von Patienten mit Friedrich-Ataxie (FRDA) abgeleitet wurden.

3. Verfahren gemäß Anspruch 1 oder 2, **dadurch gekennzeichnet, dass** in Schritt c) der zelluläre Glutathiongehalt durch Inhibieren der *de-novo-*Synthese von Glutathion reduziert wird.

4. Verfahren gemäß Anspruch 3, **dadurch gekennzeichnet, dass** der zelluläre Glutathiongehalt durch die Zugabe eines Inhibitors der γ-Glutamylcysteinsynthetase, vorzugsweise BSO (L-Buthionin-(S,R)-sulfoximin), reduziert wird.

5. Verfahren gemäß einem der Ansprüche 1 bis 4, **dadurch gekennzeichnet, dass** es sich bei der Reaktion in Schritt e) um eine erhöhte Permeabilität der Plasmamembran und/oder Zelltod handelt.

6. Verfahren gemäß einem der Ansprüche 1 bis 5, **dadurch gekennzeichnet, dass** die Reaktion in Schritt e) mit der Reaktion von Kontrollzellen mit normaler Frataxin-Expression und/oder normalem zellulärem Glutathiongehalt und/oder unter mit Selen ergänzten Inkubationsbedingungen auf die Kandidatensubstanz verglichen wird.

7. Verfahren gemäß einem der Ansprüche 1 bis 6, **dadurch gekennzeichnet, dass** die Reaktion in Schritt e) mit der Reaktion von Kontrollzellen mit reduzierter Frataxin-Expression und reduziertem zellulärem Glutathiongehalt, die in Selen-beschränktem Medium gewachsen sind, auf eine bekannte wirksame Kandidatensubstanz verglichen wird, vorzugsweise mit der Reaktion von FRDA-Fibroblasten, deren zellulärer Glutathiongehalt reduziert ist, auf Idebenon (6-(10-Hydroxydecyl)-2,3-dimethoxy-5-methyl-1,4-benzochinon) oder Ebselen (2-Phenyl-1,2-benziso-selenazol-3-(2H)-on), verglichen wird.

8. Verwendung eines GPX-Mimetikums zur Herstellung eines Medikaments zur Behandlung der Friedreich-Ataxie.

9. Verwendung gemäß Anspruch 8, **dadurch gekennzeichnet, dass** es sich bei dem GPX-Mimetikum um Ebselen (2-Phenyl-1,2-benzisoselenazol-3-(2H)-on) handelt.

10. Verwendung von GPX-Mimetika oder GPX-Mimetika in Kombination mit Idebenon (6-(10-Hydroxydecyl)-2,3-dimethoxy-5-methyl-1,4-benzochinon) zur Herstellung eines Medikaments zur Behandlung der Friedreich-Ataxie.

11. Verwendung gemäß Anspruch 8 oder 10, wobei niedermolekulare GPX-Mimetika, vorzugsweise niedermolekulare Mono- oder Diselen-Mimetika verwendet werden.

12. Verwendung gemäß Anspruch 11, wobei eine Diselenverbindung der allgemeinen Formel I, 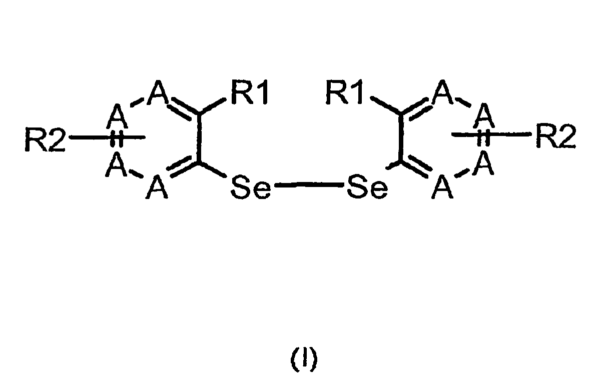 oder Formel II 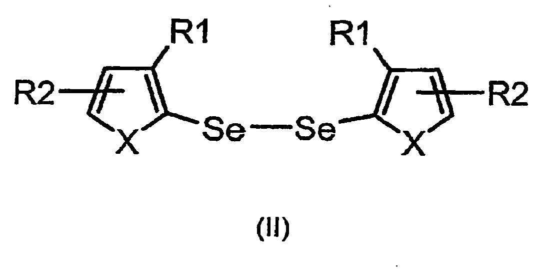 verwendet wird, wobei
A in jedem Fall unabhängig für jeden aromatischen Substituenten (a) C für alle Positionen oder (b) ein N und C für alle anderen Positionen des aromatischen Substituenten bezeichnet,
X in jedem Fall unabhängig für jeden aromatischen Substituenten S, O, NH, NR₄ bezeichnet, wobei R₄ ein lineares oder verzweigtes, gesättigtes oder ungesättiges C₁₋₁₀-Alkyl bezeichnet,
R₁ in jedem Fall unabhängig für jeden aromatischen Substituenten ein Wasserstoffatom, einen primären oder sekundären, linearen oder verzweigten, gesättigten oder ungesättigten C₁₋₆-Alkohol, einen primären oder sekundären, linearen oder verzweigten, gesättigten oder ungesättigten C₁₋₆-Ether, ein primäres, sekundäres oder tertiäres, lineares oder verzweigtes oder zyklisches, gesättigtes oder ungesättigtes C₁₋₈-Amin, einen Alkyl-substituierten C₁₋₆-Harnstoff, oder ein Alkyl- und/oder Aryl-substituiertes Imidazolin bezeichnet,
R₂ in jedem Fall unabhängig für jeden aromatischen Substituenten ein Wasserstoffatom, ein primäres oder sekundäres, lineares oder verzweigtes, gesättigtes oder ungesättigtes C₁₋₆-Alkyl, einen primären oder sekundären; linearen oder verzweigten, gesättigten oder ungesättigten C₁₋₆-Ether oder ein Nitro, Trifluormethyl, Sulfo oder Halogen bezeichnet,
und ihre Diastereomere oder Enantiomere und pharmazeutisch verträgliche Salze davon.

13. Verwendung gemäß Anspruch 11, wobei die Monoselenverbindung die allgemeine Formel III 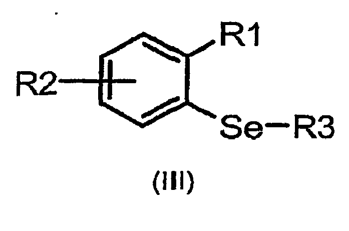 hat, wobei
R₁ einen primären oder sekundären, linearen oder verzweigten, gesättigten oder ungesättigten C₁₋₆-Alkohol, einen primären oder sekundären, linearen oder verzweigten, gesättigten oder ungesättigten C₁₋₆-Ether, ein primäres, sekundäres oder tertiäres, lineares oder verzweigtes oder zyklisches, gesättigtes oder ungesättigtes C₁₋₈-Amin, einen Alkyl-substituierten C₁₋₆-Harnstoff, oder ein Alkyl- und/oder Aryl-substituiertes Imidazolin bezeichnet,
R₂ ein Wasserstoffatom, ein primäres oder sekundäres, lineares oder verzweigtes, gesättigtes oder ungesättigtes C₁₋₆-Alkyl, einen primären oder sekundären, linearen oder verzweigten, gesättigten oder ungesättigten C₁₋₆-Ether oder zyklischen Ether, oder ein Nitro, Sulfo, Trifluormethyl, oder Halogen bezeichnet,
R₃ einen primären oder sekundären, linearen oder verzweigten, gesättigten oder ungesättigten, substituierten oder unsusbstituierten C₁₋₆-Alkohol, - nicht zyklischen oder zyklischen Ether bezeichnet,
und ihre Diastereomere oder Enantiomere und pharmazeutisch verträgliche Salze davon.

14. Verwendung einer Selenverbindung gemäß Anspruch 12 oder 13, wobei R₁ einen sekundären C₁₋₆-Alkohol, einen sekundären C₁₋₆-Ether, ein sekundäres oder tertiäres, lineares oder zyklisches C₁₋₈-Amin, einen 1,1-Di-C₁₋₆-alkyl-3-C₁₋₆₋alk-1-ylharnstoff oder ein 1,3-Di-C₁₋₆-alkyl-5-arylimidazolin, vorzugsweise einen sekundären C₁₋₄-Alkohol, einen sekundären C₁₋₄-Ether, ein sekundäres oder tertiäres, lineares oder zyklisches C₁₋₆-Amin, oder ein 1,3-Di-C₁₋₃-alkyl-5-arylimidazolin, stärker bevorzugt Propan-2-ol, 1-Hydroxypropyl, 1-Ethoxyethyl, 1,3-Dimethyl-5-phenylimidazolidin-4-yl, 1-Hydroxy-2,2-dimethylpropyl, 1-Hydroxybutyl, 1-(Dimethylamino)-ethyl oder 1-Pyrrolidon-1-yl-eth-1-yl bezeichnet.

15. Verwendung einer Selenverbindung gemäß einem der Ansprüche 12 bis 14, wobei R₂ ein Wasserstoffatom, ein primäres oder sekundäres, lineares oder verzweigtes, gesättigtes oder ungesättigtes C₁₋₄Alkyl, einen primären oder sekundären, linearen oder verzweigten, gesättigten oder ungesättigten C₁₋₄₋Ether oder ein Nitro, Trifluormethyl oder Halogen, vorzugsweise ein Wasserstoffatom, ein primäres oder sekundäres, lineares oder verzweigtes, gesättigtes C₁₋₄-Alkyl; einen primären, linearen, gesättigten C₁₋₄-Ether oder ein Nitro, Trifluormethyl oder Halogen, stärker bevorzugt ein tert-Butyl, ein Methyl, ein Nitro oder ein Methoxy, ein Chlor-, ein Brom-, ein Fluoratom oder ein Trifluormethyl bezeichnet.

16. Verwendung einer Selenverbindung gemäß Anspruch 13 bis 15, wobei R₃ einen primären oder sekundären, linearen oder verzweigten, gesättigten, substituierten oder unsubstituierten C₁₋₃-Alkohol oder nicht zyklischen C₁₋₃-Ether, vorzugsweise einen Phenyl-substituierten primären oder sekundären gesättigten C₁₋₃-Alkohol oder C₁₋₃-Ether und stärker bevorzugt ein 2-Hydroxy-1-phenylethyl, ein 2-Methoxy-2-phenylethyl bezeichnet.

17. Verwendung einer Selenverbindung gemäß Anspruch 12, wobei R₄ ein lineares oder verzweigtes, gesättigtes oder ungesättigtes C₁₋₄-Alkyl, vorzugsweise ein lineares oder verzweigtes, gesättigtes C₁₋₄-Alkyl und stärker bevorzugt ein Methyl, Ethyl oder Isopropyl bezeichnet.

18. Verwendung einer Selenverbindung gemäß den Ansprüchen 12, 14 oder 15, wobei der A umfassende aromatische Substituent ein Phenyl- oder ein 2-Pyridil-Substituent ist.

19. Verwendung einer Selenverbindung gemäß den Ansprüchen 12, 14 oder 15, wobei X NH oder O, vorzugsweise NH bezeichnet.

20. Verwendung einer Bis[2-[1-(C₁₋₆-alkylamino)-C₁₋₆-alkyl]ferrocenyl]-diselenid-Verbindung zur Herstellung eines Medikaments für die Behandlung der Friedreich-Ataxie.

21. Verwendung eines GPX-Mimetikums gemäß einem der Ansprüche 8 und 10 bis 20, wobei das Mimetikum ausgewählt ist aus Bis[2-(propan-2-ol)-phenyl]-diselenid, (S,S)-Bis[2-(1-hydroxypropyl)-5-tert-butyl-phenyl]-diselenid, (S,S)-Bis[3-(1-ethoxyethyl)-pyridin-2]diselenid, 1-[2-(2-Hydroxy-(S)-1-phenylethyl selenyl)-phenyl]-propan-(R)-1-ol, 1-[2-(2-Hydroxy-(S)-1-phenylethylselenyl)-phenyl]-propan-(S)-1-ol, (S,S)-Bis[2-(1-hydroxypropyl)-6-methyl-phenyl]-diselenid, (S,S)-Bis[2-(1-hydroxypropyl)-4-nitro-phenyl]-diselenid, (S)-1-[3-Methoxy-2 -(2 -phenyl-tetrahydrofuran-3-yl-selenyl)-phenyl]-ethanol, Bis[2-(1,3-Dimethyl-(S)-5-phenyl-imidazolidin-(S)-4-yl)-phenyl]-diselenid, (Bis[2-(1-hydroxy-2,2-dimethyl-propyl-phenyl]-diselenid, Bis[4-methoxy-phenyl]-diselenid, (Bis[2-(1-hydroxy-butyl-phenyl]-diselenid, [R,S;R,S]-Bis[2-[1-(dimethylamino)-ethyl]ferrocenyl]-diselenid, (R,R)-Bis[2-(1,1-dimethyl-3-eth-1-yl-harnstoff)-phenyl]-diselenid, (R,R)-Bis[2-(1-dimethylamino-eth-1-yl)-phenyl]-diselenid, (R,R)-Bis[2-(1-pyrrolidin-1-yl-eth-1-yl)-phenyl]-diselenid.

22. Verwendung gemäß einem der Ansprüche 8 bis 21, wobei die Selenverbindung mit Radikalfängern und/oder Antioxidantien, vorzugsweise Coenzym Q10 oder Derivaten davon, N-Acetylcystein und/oder Vitamin E oder Derivaten davon kombiniert wird.

23. Verwendung gemäß einem der Ansprüche 8 bis 22 in Kombination mit Buspiron, Amantadin-Salzen, Idebenon und/oder neurotrophen Faktoren, vorzugsweise Insulin-ähnlicher Wachstumsfaktor I (IGF-I).

24. Verwendung gemäß einem der Ansprüche 11 bis 23 in Kombination mit Selen.

25. Verwendung von Zellen mit reduzierter Frataxin-Expression und einem reduzierten zellulären Glutathiongehalt zum Identifizieren und/oder Validieren von Kandidatensubstanzen zur Behandlung der Friedreich-Ataxie (FRDA), vorzugsweise Zellen mit einem reduzierten zellulären Glutathiongehalt, die von Patienten mit Friedreich-Ataxie (FRDA) abgeleitet oder isoliert wurden.

26. Verwendung gemäß Anspruch 25, **dadurch gekennzeichnet, dass** ein Inhibitor der γ-Glutamylcysteinsynthetase, vorzugsweise BSO (L-Buthionin-(S,R)-sulfoximin), zu den Zellen gegeben wird und die Zellen in Selen-beschränktem Medium gezüchtet werden.

## Revendications

1. Procédé pour identifier et/ou valider des substances candidates pour le traitement de l'ataxie de Friedreich, comprenant les étapes consistant :
a) à fournir des cellules ayant une expression réduite de frataxine,
b) à incuber les cellules de l'étape a) dans un milieu restreint en sélénium,
c) à réduire la teneur en glutathion cellulaire des cellules de b),
d) à mettre en contact les cellules de l'étape c) avec une substance candidate, et
e) à évaluer la réponse des cellules de l'étape d),
dans lequel les étapes b), c) et d) peuvent aussi être réalisées dans tout autre ordre que b), c) et d), l'ordre b), c) et d) étant préféré.

2. Procédé selon la revendication 1, **caractérisé en ce que** les cellules de l'étape a) sont des cellules isolées ou issues de patients d'ataxie de Friedreich (FRDA), de préférence des cellules fibroblastes issues de patients d'ataxie de Friedreich (FRDA).

3. Procédé selon la revendication 1 ou 2, **caractérisé en ce que** dans l'étape c), la teneur en glutathion cellulaire est réduite en inhibant la synthèse *de novo* du glutathion.

4. Procédé selon la revendication 3, **caractérisé en ce que** la teneur en glutathion cellulaire est réduite par l'addition d'un inhibiteur de la γ-glutamyl cystéine synthétase, de préférence la BSO (L-buthionine-(S,R)-sulfoximine).

5. Procédé selon l'une quelconque des revendications 1 à 4, **caractérisé en ce que** ladite réponse dans l'étape e) est une augmentation de perméabilité de la membrane plasmatique et/ou une mort cellulaire.

6. Procédé selon l'une quelconque des revendications 1 à 5, **caractérisé en ce que** ladite réponse dans l'étape e) est comparée à la réponse de cellules témoins ayant une expression normale de frataxine et/ou une teneur normale en glutathion cellulaire et/ou dans des conditions d'incubation supplémentée en sélénium, à ladite substance candidate.

7. Procédé selon l'une quelconque des revendications 1 à 6, **caractérisé en ce que** ladite réponse dans l'étape e) est comparée à la réponse de cellules témoins ayant une expression réduite de frataxine et une teneur réduite en glutathion cellulaire, développées dans un milieu restreint en sélénium, à une substance candidate efficace connue, de préférence comparée à la réponse de fibroblastes-FRDA, qui sont réduits en teneur en glutathion cellulaire, à l'Idébénone (6-(10-hydroxydécyl)-2,3-diméthoxy-5-méthyl-1,4-benzoquinone) ou l'Ebselen (2-phényl-1,2-benziso-sélénazol-3-(2H)-one).

8. Utilisation d'un mimétique de GPX pour la préparation d'un médicament pour le traitement de l'ataxie de Friedreich.

9. Utilisation selon la revendication 8, **caractérisée en ce que** le mimétique de GPX est l'Ebselen (2-phényl-1,2-benzisosélénazol-3-(2H)-one).

10. Utilisation de mimétiques de GPX ou de mimétiques de GPX combinés avec de l'Idébénone (6-(10-hydroxydécyl)-2,3-diméthoxy-5-méthyl-1,4-benzoquinone) pour la préparation d'un médicament pour le traitement de l'ataxie de Friedreich.

11. Utilisation selon la revendication 8 ou 10, dans laquelle des mimétiques de GPX à petite molécule, de préférence des mimétiques à petite molécule mono- ou diséléno, sont utilisés.

12. Utilisation selon la revendication 11, dans laquelle un composé diséléno de formule générale I, 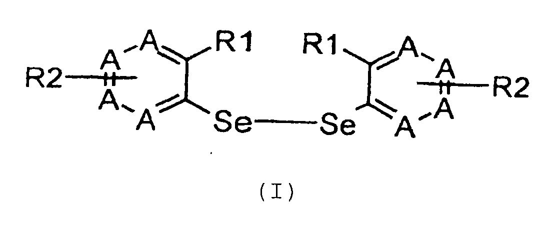 ou de formule II 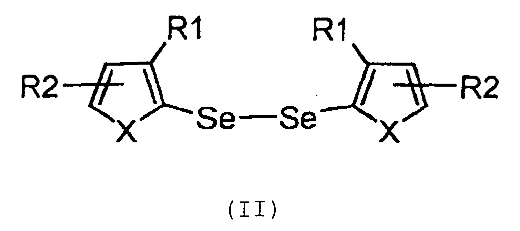 est utilisé, où
A représente, dans chaque cas indépendamment pour chaque substituant aromatique, (a) C pour toutes les positions ou (b) un N et C pour toutes les autres positions du substituant aromatique,
X représente, dans chaque cas indépendamment pour chaque substituant aromatique, S, O, NH, NR₄ où R₄ représente un alkyle en C₁₋₁₀ linéaire ou ramifié, saturé ou insaturé,
R₁ représente, dans chaque cas indépendamment pour chaque substituant aromatique, un hydrogène, un alcool en C₁₋₆ primaire ou secondaire, linéaire ou ramifié, saturé ou insaturé, un éther en C₁₋₆ primaire ou secondaire, linéaire ou ramifié, saturé ou insaturé, une amine en C₁₋₈ primaire, secondaire ou tertiaire, linéaire ou ramifiée ou cyclique, saturée ou insaturée, une urée en C₁₋₆ à substitution alkyle, ou une imidazoline à substitution alkyle et/ou aryle,
R₂ représente, dans chaque cas indépendamment pour chaque substituant aromatique, un hydrogène, un groupe alkyle en C₁₋₆ primaire ou secondaire, linéaire ou ramifié, saturé ou insaturé, un éther en C₁₋₆ primaire ou secondaire, linéaire ou ramifié, saturé ou insaturé, ou un nitro, trifluorométhyle, sulfo ou halogéno,
et ses diastéréomères ou énantiomères et les sels pharmaceutiquement acceptables de ceux-ci.

13. Utilisation selon la revendication 11, dans laquelle le composé monoséléno a la formule générale III, 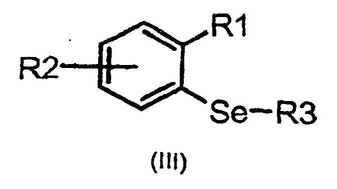 dans laquelle
R₁ représente un alcool en C₁₋₆ primaire ou secondaire, linéaire ou ramifié, saturé ou insaturé, un éther en C₁₋₆ primaire ou secondaire, linéaire ou ramifié, saturé ou insaturé, une amine en C₁₋₈ primaire, secondaire ou tertiaire, linéaire ou ramifiée ou cyclique, saturée ou insaturée, une urée en C₁₋₆ à substitution alkyle, ou une imidazoline à substitution alkyle et/ou aryle,
R₂ représente un hydrogène, un alkyle en C₁₋₆ primaire ou secondaire, linéaire ou ramifié, saturé ou insaturé, un éther en C₁₋₆ primaire ou secondaire, linéaire ou ramifié, saturé ou insaturé ou un éther cyclique, ou un nitro, sulfo, trifluorométhyle ou halogéno,
R₃ représente un alcool en C₁₋₆ primaire ou secondaire, linéaire ou ramifié, saturé ou insaturé, substitué ou non substitué, un éther non cyclique ou cyclique,
et ses diastéréomères ou énantiomères et les sels pharmaceutiquement acceptables de ceux-ci.

14. Utilisation d'un composé séléno selon la revendication 12 ou 13, dans laquelle R₁ représente un alcool en C₁₋₆ secondaire, un éther en C₁₋₆ secondaire, une amine en C₁₋₈ secondaire ou tertiaire, linéaire ou cyclique, une 1,1-di-alkyl en C₁₋₆-3-alk-1-yl en C₁₋₆-urée ou une 1,3-di-alkyl en C₁₋₆-5-aryl imidazoline, de préférence un alcool en C₁₋₄ secondaire, un éther en C₁₋₄ secondaire, une amine en C₁₋₆ secondaire ou tertiaire, linéaire ou cyclique, ou une 1,3-di-alkyl en C₁₋₃-5-aryl imidazoline, de préférence encore le propan-2-ol, un groupe 1-hydroxypropyle, 1-éthoxyéthyle, 1,3-diméthyl-5-phényl-imidazolidin-4-yle., 1-hydroxy-2,2-diméthyl-propyle, 1-hydroxybutyle, 1-(diméthylamino)-éthyle ou 1-pyrrolidine-1-yl-éth-1-yle.

15. Utilisation d'un composé séléno selon l'une quelconque des revendications 12 à 14, dans laquelle R₂ représente un hydrogène, un alkyle en C₁₋₄ primaire ou secondaire, linéaire ou ramifié, saturé ou insaturé, un éther en C₁₋₄ primaire ou secondaire, linéaire ou ramifié, saturé ou insaturé, ou un nitro, trifluorométhyle ou halogéno, de préférence un hydrogène, un alkyle en C₁₋₄ saturé, primaire ou secondaire, linéaire ou ramifié, un éther en C₁₋₄ primaire, linéaire, saturé, ou un nitro, trifluorométhyle ou halogéno, de préférence encore un tert-butyle, méthyle, nitro ou un méthoxy, un chloro, un bromo, un fluoro ou un trifluorométhyle.

16. Utilisation d'un composé séléno selon les revendications 13 à 15, dans laquelle R₃ représente un alcool en C₁₋₃ primaire ou secondaire, linéaire ou ramifié, saturé, substitué ou non substitué, ou un éther en C₁₋₃ non cyclique, de préférence un alcool en C₁₋₃ ou éther en C₁₋₃ saturé primaire ou secondaire à substitution phényle, et de préférence encore un groupe 2-hydroxy-1-phényléthyle, un groupe 2-méthoxy-2-phényléthyle.

17. Utilisation d'un composé séléno selon la revendication 12, dans laquelle R₄ représente un alkyle en C₁₋₄ linéaire ou ramifié, saturé ou insaturé, de préférence un alkyle en C₁₋₄ saturé, linéaire ou ramifié, et de préférence encore un méthyle, éthyle ou isopropyle.

18. Utilisation d'un composé séléno selon les revendications 12, 14 ou 15, dans laquelle le substituant aromatique comprenant A est un substituant phényle ou 2-pyridyle.

19. Utilisation d'un composé séléno selon les revendications 12, 14 ou 15, dans laquelle X représente NH ou O, de préférence NH.

20. Utilisation d'un composé bis[2-[1-(alkyl en C₁₋₆-amino) -alkyl en C₁₋₆] ferrocényl] diséléniure pour la préparation d'un médicament pour le traitement de l'ataxie de Friedreich.

21. Utilisation d'un mimétique de GPX selon l'une quelconque des revendications 8, et 10 à 20, dans laquelle le mimétique est choisi parmi le bis[2-(propan-2-ol)-phényl]-diséléniure, le (S,S)-bis[2-(1-hydroxypropyl)-5-tert-butyl-phényl]-diséléniure, le (S,S)-bis[3-(1-éthoxyéthyl)-pyridine-2]-diséléniure, le 1-[2-(2-hydroxy-(S)-1-phényl éthyl sélényl)-phényl]-propan-(R)-1-ol, le 1-[2-(2-hydroxy-(S)-1-phényl éthyl sélényl)-phényl]-propan-(S)-1-ol, le (S,S)-bis[2-(1-hydroxypropyl)-6-méthyl-phényl]-diséléniure, le (S,S)-bis[2-(1-hydroxypropyl)-4-nitro-phényl]-diséléniure, le (S)-1-[3-méthoxy 2-(2-phényl-tétrahydrofuran-3-yl-sélényl)-phényl]-éthanol, le bis[2-(1,3-diméthyl-(S)-5-phényl-imidazolidin-(S)-4-yl)-phényl]-diséléniure, le (bis[2-(1-hydroxy-2,2-diméthyl-propyl-phényl]-diséléniure, le bis[4-méthoxy-phényl]-diséléniure, le (bis[2-(1-hydroxy-butyl-phényl]-diséléniure, le [R,S;R,S]-bis[2-[1-(diméthylamino)-éthyl]ferrocényl]-diséléniure, le (R,R)-bis[2-(1,1-diméthyl-3-éth-1-yl-urée)-phényl]-diséléniure, le (R,R)-bis[2-(1-diméthylamino-éth-1-yl)-phényl]-diséléniure, le (R,R)-bis[2-(1-pyrrolidine-1-yl-éth-1-yl)-phényl]-diséléniure.

22. Utilisation selon l'une quelconque des revendications 8 à 21, dans laquelle le composé séléno est combiné avec des piégeurs de radicaux libres et/ou des antioxydants, de préférence la coenzyme Q10 ou des dérivés de celle-ci, la N-acétyl cystéine, et/ou la vitamine E ou des dérivés de celle-ci.

23. Utilisation selon l'une quelconque des revendications 8 à 22, en combinaison avec de la buspirone, des sels d'amantadine, de l'Idébénone et/ou des facteurs neurotrophiques, de préférence le facteur de croissance semblable à l'insuline I (IGF-1).

24. Utilisation selon l'une quelconque des revendications 11 à 23, en combinaison avec du sélénium.

25. Utilisation de cellules ayant une expression réduite de frataxine et une teneur réduite en glutathion cellulaire pour identifier et/ou valider des substances candidates pour le traitement de l'ataxie de Friedreich (FRDA), de préférence des cellules ayant une teneur réduite en glutathion cellulaire dérivées ou isolées de patients d'ataxie de Friedreich (FRDA).

26. Utilisation selon la revendication 25, **caractérisée en ce qu'**un inhibiteur de la γ-glutamyl cystéine synthétase, de préférence la BSO (L-buthionine-(S,R)-sulfoximine), est ajouté auxdites cellules et lesdites cellules sont cultivées dans un milieu restreint en sélénium.
